## Europäisches Patentamt
## European Patent Office
## Office européen des brevets

(19)

(11) Numéro de publication: **0 030 760**
**A1**

(12) **DEMANDE DE BREVET EUROPEEN**

(21) Numéro de dépôt: **80201148.6**

(22) Date de dépôt: **04.12.80**

(51) Int. Cl.³: **A 61 M 1/03**
**G 05 D 9/12**

(30) Priorité: **12.12.79 FR 7930666**

(43) Date de publication de la demande:
**24.06.81 Bulletin 81/25**

(84) Etats Contractants Désignés:
**AT BE CH DE FR GB IT LI LU NL SE**

(71) Demandeur: **INSTITUT NATIONAL DE LA SANTE ET DE LA RECHERCHE MEDICALE (INSERM)**
**101, rue de Tolbiac**
**F-75654 Paris Cedex 13(FR)**

(72) Inventeur: **Barthelemy, Robert**
**Les Dames - Auzeville**
**F-31320 Castanet-Tolosan(FR)**

(72) Inventeur: **Brieussel, Jean-Marie**
**119, rue du Férétra**
**F-31400 Toulouse(FR)**

(72) Inventeur: **Couzi, Hugues**
**10, rue du Commandeur Cazeneuve**
**F-31400 Toulouse(FR)**

(72) Inventeur: **Morucci, Jean-Pierre**
**Route de Rouquettes - Pin-Justaret**
**F-31120 Portet-sur-Garonne(FR)**

(74) Mandataire: **Barre, Philippe**
**Cabinet Barre-Gatti-Laforque 93-95 rue des Amidonniers**
**F-31069 Toulouse Cédex(FR)**

(54) Dispositif de régulation du débit de sang délivré par un système de circulation extracorporelle, moyen de détection d'une quantité de liquide et élément inductif utilisé dans ce dispositif.

(57) Le dispositif de l'invention comprend des moyens de détection I de la quantité de sang contenue dans l'unité d'oxygénation du système de circulation extracorporelle et un ensemble électronique de traitement du signal issu de ces moyens de détection en vue d'asservir les moyens de pompage du système de circulation extracorporelle ; l'ensemble électronique de traitement comprend en particulier une mémoire de consigne 3 associée à une logique de blocage 4 qui permet de figer, à un instant donné, le signal détecté reçu dans le mémoire en vue d'afficher une valeur consigne par rapport à laquelle la régulation de la quantité de sang s'effectue.

L'invention apporte un perfectionnement au système de circulation extracorporelle en assurant une régulation automatique du débit de sang délivré à chaque instant vers le malade, sans risque d'envoi vers celui-ci de bulles gazeuses.

./...

Fig.1

0030760

DISPOSITIF ET MOYENS POUR LA REGULATION DU DEBIT DE SANG DELI-VRE PAR UN SYSTEME DE CIRCULATION EXTRACORPORELLE.

L'invention concerne un dispositif de régulation du débit de sang délivré vers un malade par un système de circulation extracorporelle ayant pour rôle d'assurer la fonction du coeur et du poumon pendant leur exclusion au cours d'opérations chirurgicales intra-cardiaques.

On sait que les systèmes de circulation extracorporelles sont destinés à dériver le sang d'un malade avant qu'il ne pénètre dans le coeur et à l'injecter, après oxygénation et épuration du $CO_2$, au niveau de l'aorte de façon à assurer la vie des tissus pendant la mise hors circuit du coeur. Ces systèmes comprennent de façon classique une unité d'oxygénation et des moyens de pompage permettant la réinjection du sang.

Il est essentiel, lors de la réinjection, d'éviter l'envoi vers le malade de bulles gazeuses susceptibles de provoquer une embolie : dans les systèmes actuels qui sont en exploitation, il est nécessaire qu'un opérateur surveille en permanence le niveau de sang dans le système et ajuste, de temps à autre, la vitesse des moyens de pompage pour éviter que le système se vide de sang. Cette tâche constitue une servitude contraignante et demeure soumise aux risques d'erreurs humaines toujours possibles. En outre, il arrive que l'opérateur assure des corrections brutales qui peuvent présenter un caractère traumatisant pour le malade.

Il est important de remarquer que le volume de sang à l'extérieur du malade doit être aussi réduit que possible (économie de sang) ; dans les systèmes connus actuellement en exploitation, ce volume est de l'ordre de 2 litres et le débit de circulation est de l'ordre de 5 litres/minute : dans ces conditions, le système peut se vider de sang en une vingtaine de secondes, ce qui met en évidence la nécessité d'une surveillance très attentive et la gravité des conséquences d'une inattention ou d'une fausse manoeuvre.

Par ailleurs, certains documents antérieurs décrivent des dispositifs ayant pour objectif d'augmenter la sécurité de fonctionnement de ces systèmes de circulation extracorporelle. Ainsi par exemple, le brevet US n° 3851181 décrit un dispositif de détection infrarouge qui est monté sur un oxygénateur
à bulles d'un système de circulation extracorporelle en vue
d'engendrer une alarme de niveau. Ce dispositif avertit l'opérateur lorsque le niveau de sang devient anormal, mais celui-ci
doit prendre lui-même les mesures nécessaires ; de plus, un tel
dispositif n'écarte pas les risques de traumatisme provenant de
variations brusques du débit de sang ; au surplus, sa conception
le rend uniquement applicable dans le cas des oxygénateurs à
bulles.

Dans un autre domaine, celui de la dialyse rénale, le
brevet FR publié sous le n° 2053869 décrit un dispositif de régulation agissant sur les moyens de pompage pour tendre à ajuster un niveau de sang à un niveau consigne. Toutefois, en pratique, un tel dispositif est d'utilisation très délicate du
fait que le signal de consigne est élaboré par une méthode de
"faux zéro" sans tenir compte des caractéristiques du système
(caractéristiques du système de circulation extracorporelle et
en particulier de son oxygénation et de ses moyens de pompage,
et caractéristiques propres du dispositif de régulation) ; dans
chaque cas, un tel dispositif nécessite d'effectuer un étalonnage préalable précis pour pouvoir fixer le niveau de consigne
à la valeur désirée et ce travail préalable est relativement
complexe. De plus, ce type de dispositif ne fournit pas les sécurités souhaitables évoquées plus haut (suppression des à-coups,
suppression de l'influence des fausses manoeuvres).

La présente invention vise à remédier aux défauts des
systèmes actuels de circulation extracorporelle, en fournissant
un dispositif automatique de régulation du débit de sang délivré vers un malade.

Un objectif de l'invention est en particulier de régulariser en permanence et automatiquement la quantité de sang
dans le système de façon à écarter totalement les risques d'insufflation de bulles gazeuses vers le malade.

Un autre objectif est d'éliminer toute variation brutale du débit injecté vers le malade.

Un autre objectif est d'autoriser, sans risque, une réduction de la quantité totale de sang contenue dans le système
de circulation extracorporelle de façon à économiser cette substance et à réduire le caractère traumatisant de l'opération.

Un autre objectif essentiel de l'invention est de fournir un dispositif de régulation universel, susceptible d'être utilisé pour tout système de circulation extracorporelle, quel que soit le type de l'unité d'oxygénation de ce système et quelles que soient les caractéristiques des moyens de pompage et ce, sans nécessité d'un étalonnage préalable pour régler la valeur consigne autour de laquelle doit s'effectuer la régulation de la quantité de sang.

Un autre objectif est de fournir un dispositif de régulation susceptible de fonctionner en régime manuel ou en régime automatique, sans risque de variation brusque du débit de sang pendant les changements de régime.

Un autre objectif est de fournir un dispositif de régulation ne présentant aucun risque de traumatisme du sang contenu dans le système de circulation extracorporelle.

Un autre objectif est de fournir un dispositif de régulation ayant un fonctionnement indifférent à l'environnement et qui soit en particulier immunisé à l'égard de l'influence d'éventuels champs électriques extérieurs.

Un autre objectif est de fournir un dispositif de régulation susceptible de s'appliquer, aussi bien sur les systèmes à oxygénateur à bulles, que sur les systèmes à oxygénateur à membrane.

Le dispositif de régulation visé par l'invention est destiné à assurer la régulation du débit de sang délivré vers un malade par un système de circulation extracorporelle essentiellement composé :

. de moyens de conduite du sang comportant un conduit amont de prélèvement du sang du malade et un conduit aval d'injection du sang vers le malade,

.de moyens de pompage engendrant une circulation du sang du conduit amont de prélèvement vers le conduit aval d'injection,

. et d'une unité d'oxygénation du sang placée entre le conduit amont de prélèvement et le conduit aval d'injection.

Selon la présente invention, ce dispositif de régulation comprend :

4

. des moyens de détection de la quantité de sang contenue à chaque instant dans l'unité d'oxygénation, adaptés pour
délivrer un signal électrique représentatif à chaque instant de
cette quantité de sang,

. une mémoire, dite de consigne, recevant le signal
issu des moyens de détection et associée à une logique de blocage permettant de figer à un instant donné le signal reçu dans
la mémoire,

. un comparateur, recevant, d'une part, le signal issu
des moyens de détection, d'autre part, le signal issu de la mémoire de consigne et adapté pour délivrer un signal d'écart représentatif de la différence entre les signaux reçus,

. un ensemble potentiométrique adapté pour délivrer un
signal de vitesse correspondant à une vitesse initiale désirée
des moyens de pompage,

. un sommateur recevant le signal d'écart issu du comparateur et le signal de vitesse issu de l'ensemble potentiométrique et adapté pour délivrer vers les moyens de pompage un
signal d'asservissement approprié pour accélérer lesdits moyens
de pompage en cas d'augmentation de la quantité de sang détectée et pour ralentir ces moyens de pompage en cas de baisse de
cette quantité de sang.

La mémoire de consigne conserve la valeur consigne représentative de la quantité de sang au moment de son blocage,
et c'est par rapport à cette valeur que le comparateur élabore
un signal d'écart apte à moduler à travers le sommateur le
signal de vitesse initiale. Par exemple, si les moyens de détection détectent une quantité de sang inférieure à la valeur
consigne, le sommateur élabore un signal modulé par rapport
au signal de vitesse initiale, qui ralentit la pompe jusqu'à
ce que le système de circulation extracorporelle contienne à
nouveau une quantité de sang égale à la quantité initiale
enregistrée au moment du blocage de la mémoire. Par le choix
de l'instant du blocage, l'opérateur peut ajuster dans chaque
cas la quantité de sang consigne autour de laquelle s'effectue
la régulation, en fonction notamment de la corpulence du patient ou des contraintes médicales.

5

Cet ajustement s'effectue sans qu'il soit besoin d'effectuer un étalonnage quelconque du dispositif, quelles que soient les caractéristiques de celui-ci et celles du système de circulation extracorporelle. En effet, le signal de consigne qui est mémorisé dans la mémoire de consigne pour fournir la consigne de niveau de sang est élaboré dans le dispositif de l'invention à partir du signal issu des moyens de détection en figeant ce signal à un instant donné.

Le dispositif de régulation est avantageusement complété par des moyens d'alarme adaptés pour se déclencher dans le cas où la quantité de sang dans l'unité d'oxygénation sort d'une plage délimitée par des seuils minimum et maximum prédéterminés ; l'opérateur est ainsi averti des situations exceptionnelles où les moyens de pompage ne parviennent plus à assurer, dans leur plage normale de fonctionnement, une égalité entre le débit de sang injecté et le débit de sang prélevé.

Les moyens d'alarme sus-évoqués peuvent être doublés par des moyens d'alarme complémentaires adaptés pour se déclencher dans le cas où la vitesse des moyens de pompage atteint des seuils minimum ou maximum. L'opérateur est ainsi immédiatement averti que la régulation impose aux moyens de pompage un fonctionnement sortant de la plage normale de fonctionnement.

Les deux types d'alarmes ci-dessus décrits se combinent pour couvrir toutes les situations exceptionnelles susceptibles de se produire, et en avertir immédiatement l'opérateur qui peut intervenir pour faire face à la situation.

Il faut noter que, même en l'absence d'intervention dans ces situations exceptionnelles, le système ne peut injecter de l'air vers le malade, puisque le dispositif de régulation aura provoqué l'arrêt de la pompe avant que le système soit entièrement vidé de sang. Tout au plus, le malade peut ne pas être irrigué pendant quelques instants, ce qui n'est pas préjudiciable.

Le dispositif de régulation conforme à l'invention peut être appliqué dans le cas d'un système de circulation extracorporelle dont l'unité d'oxygénation est composée d'un oxygénateur à bulles. Dans ce cas, les moyens de détection comprennent, de préférence, un tube auxiliaire en matériau isolant électrique monté en vase communicant sur l'oxygénateur, un condensateur ayant des armatures situées en regard de part et d'autre du tube auxiliaire, et des moyens de mesure de la capacité dudit condensateur délivrant le signal représentatif de la quantité de sang dans l'oxygénateur.

De tels moyens de détection peuvent être aisément montés sur les oxygénateurs à bulles existants ; ils ne sont pas directement en contact avec le sang et ne mettent pas en jeu des énergies risquant de développer des traumatismes sur les éléments constitutifs du sang. Notons que le tube auxilaire peut contenir du sérum physiologique de sorte que, dans ce cas, le faible champs électrique développé par les moyens de détection n'est pas appliqué directement au sang.

Le dispositif de régulation de l'invention peut également être appliqué à un système de circulation extracorporelle dont l'unité d'oxygénation est composée d'un oxygénateur à membrane et d'au moins un réservoir souple de sang disposé en amont et/ou en aval de cet oxygénateur à membrane, pour régulariser le débit de sang dans celui-ci. Dans ce cas,les moyens de détection comprennent de préférence deux éléments inductifs fixés en regard l'un de l'autre, de part et d'autre du réservoir souple à l'extérieur de celui-ci, un oscillateur électrique relié à l'un des éléments inductifs en vue de son excitation et des moyens de mesure du signal induit dans l'autre élément inductif.

Ces moyens de détection, de conception nouvelle en soi, présentent des avantages analogues à ceux décrits dans le cas des oxygénateurs à bulles.

Par ailleurs, dans les modes de réalisation ci-dessus évoqués où les moyens de détection mesurent une grandeur électrique ou électro-magnétique, ces moyens sont avantageusement associés à un circuit électronique dit d'immunité, disposés immédiatement en aval de ceux-ci en vue d'éliminer l'influence de champs parasites extérieurs, tels, par exemple, que champs créés par un bistouri électrique.

L'invention ayant été exposée dans sa forme générale, d'autres caractéristiques, buts et avantages de celle-ci se dégageront de la description qui suit en référence aux dessins annexés, lesquels en présentent des modes de réalisation préférentiels ; sur ces dessins, qui font partie intégrante de la présente description :

- la figure 1 est un schéma bloc, illustrant un dispositif de régulation conforme à l'invention associé à un système de circulation extracorporelle de type classique,

- la figure 2 est une vue schématique d'un mode de réalisation de dispositif de régulation, associé à un système de circulation extracorporelle comportant un oxygénateur à bulles,

- la figure 3 est un schéma bloc électronique montrant les moyens de détection de quantité de sang du dispositif de régulation de la figure 2,

- les figures 4, 5 et 6 sont des coupes respectivement par un plan vertical AA, par un plan horizontal BB et par un plan horizontal CC, illustrant la structure physique des moyens de détection de la figure 3,

- la figure 7 est une vue schématique d'un mode de réalisation de dispositif de régulation, associé à un système de circulation extracorporelle comportant un oxygénateur à membrane,

- la figure 8 est un schéma bloc électronique, montrant les moyens de détection de quantité de sang du dispositif de la figure 7,

- la figure 9 est une coupe partielle, axiale, illustrant la structure physique des moyens de détection de la figure 8,

- la figure 10 est un schéma bloc d'un circuit électronique d'immunité qui est avantageusement disposé à la sortie des moyens de détection sus-évoqués.

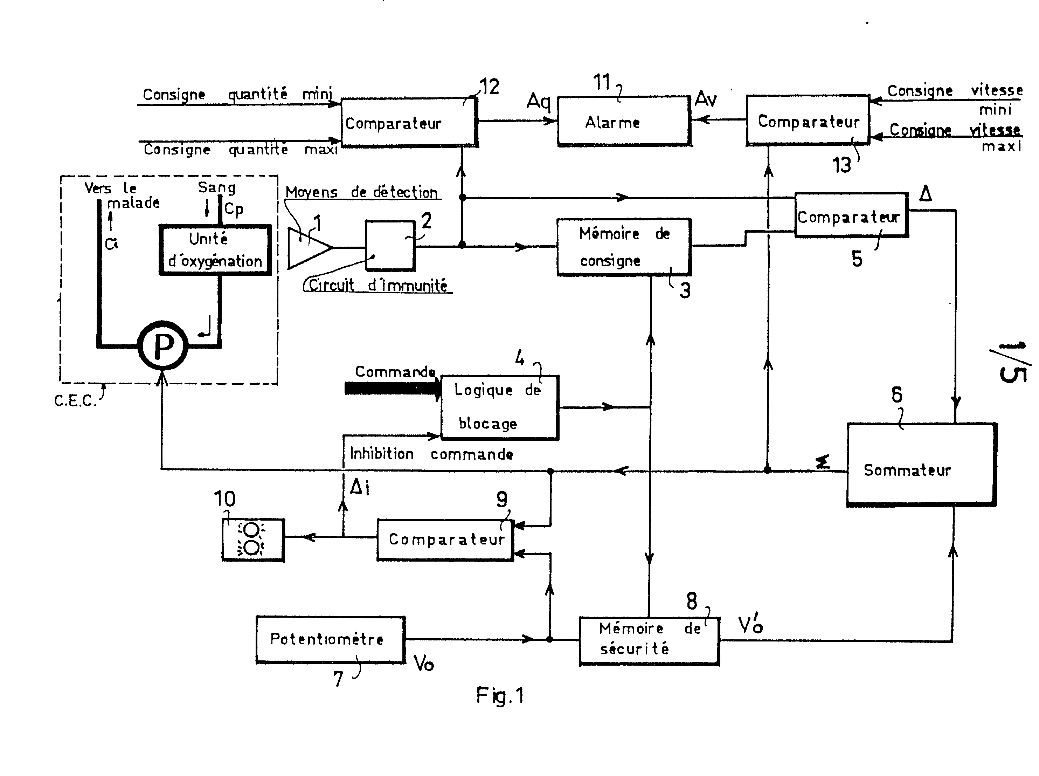

Le dispositif de régulation représenté à titre d'exemple à la figure 1 est associé à un système de circulation extracorporelle classique (désigné de façon habituelle par C.E.C.), appelé à être raccordé sur les veines caves et sur l'aorte d'un malade en vue de dériver le sang s'écoulant dans les veines caves et de le réinjecter après traitement au niveau de l'aorte. De façon classique, cette C.E.C. comprend un conduit amont de prélèvement Cp qui prélève par gravité le sang s'écoulant des veines caves, une unité d'oxygénation qui l'oxygène et le débarrasse du $CO_2$ et des moyens de pompage P qui assurent sa circulation et sa réinjection vers l'aorte par un conduit aval d'injection Ci.

Une telle C.E.C. est classique en elle-même et permet au chirurgien de travailler sur un coeur exsangue et immobile avec une irrigation des tissus du corps du malade.

Conformément à la présente invention, cette C.E.C. est équipée d'un dispositif de régulation tel que schématisé à la figure 1.

Ce dispositif de régulation comprend, d'une part, des moyens de détection 1, adaptés pour délivrer un signal électrique représentatif à chaque instant de la quantité de sang contenue dans l'unité d'oxygénation de la C.E.C., d'autre part, un ensemble électronique de traitement de ce signal. On décrira plus loin des exemples de moyens de détection, adaptés aux deux principaux types d'unités d'oxygénation actuellement utilisés.

Ces moyens de détection 1 délivrent le signal représentatif de la quantité de sang vers un circuit d'immunité 2 qui transmet intégralement ce signal en l'absence de champs électriques parasites ou, au contraire, bloque sa transmission lorsque des champs parasites apparaissent pour transmettre, pendant toute la période de blocage, un signal de niveau égal au dernier signal reçu avant l'apparition des parasites. On élimine ainsi la prise en compte de signaux parasites qui pourraient perturber dangereusement la régulation. Ce circuit d'immunité 2 est utile lorsque les moyens de détection sont de nature électronique et sont sensibles aux champs parasites. On décrira plus loin un exemple de circuit d'immunité.

Le signal issu des moyens de détection à travers le circuit d'immunité est délivré, d'une part, vers des moyens d'alarme qui seront décrits plus loin, d'autre part, vers le circuit de régulation proprement dit.

Ce dernier comprend une mémoire de consigne 3 à laquelle est associée une logique de blocage 4, permettant de figer à un instant donné le signal reçu dans ladite mémoire de consigne 3. Ce blocage peut être obtenu par une commande manuelle à 2 positions (position de blocage correspondant au régime de régulation automatique, ou position inverse correspondant au réglage manuel de la vitesse des moyens de pompage P) ; le blocage peut également être obtenu par tout autre moyen et notamment par une programmation préalable.

A la sortie de la mémoire de consigne 3, est disposé un comparateur 5 qui reçoit, d'une part, le signal issu de ladite mémoire 3, d'autre part, le signal détecté issu directement du circuit d'immunité 2. Ce comparateur délivre un signal d'écart $\Delta$ représentatif de la différence entre les signaux reçus.

En conséquence, tant que la mémoire de consigne 3 n'est pas figée, le comparateur 5 délivre un signal nul puisque les signaux délivrés à ses deux entrées sont identiques. Au contraire, lorsque la mémoire 3 est figée sur une valeur de consigne, le signal d'écart $\Delta$ prend une valeur différente de O si les moyens de détection 1 délivrent un signal représentatif de la quantité de sang, différent du signal initial enregistré dans la mémoire 3 au moment de son blocage.

Le signal d'écart $\Delta$ issu du comparateur 5 est délivré vers un sommateur 6 et sert à moduler un signal $V'_o$ d'initialisation de la vitesse des moyens de pompage P.

Le sommateur 6 délivre vers les moyens de pompage un signal modulé $\Sigma$ qui commande l'alimentation électrique de ceux-ci et impose donc une vitesse de rotation à ces moyens.

Le signal d'initialisation $V_o'$ est engendré par un ensemble potentiométrique manoeuvrable par un opérateur et qui est pourvu de sécurités éliminant les risques de brusques variations du signal $\mathcal{E}$, notamment :

. en cas de fausse manoeuvre lorsque la régulation automatique est en fonctionnement (manoeuvre accidentelle du bouton de l'ensemble potentiométrique),

. ou bien à l'instant où la régulation automatique est arrêtée en vue de revenir à un réglage manuel de la vitesse des moyens de pompage P (commande de la logique 4 pour débloquer la mémoire 3).

A cet effet, l'ensemble potentiométrique délivrant le signal $V_o'$ comprend un potentiomètre 7 pour engendrer un signal $V_o$ de valeur réglable et une mémoire de sécurité 8, recevant ce signal $V_o$ de valeur réglable, et reliée à la logique de blocage 4, en vue d'être figée en même temps que la mémoire de consigne 3 lorsque cette logique 4 est commandée.

Ainsi, la mémoire de sécurité 8 enregistre le signal de vitesse initiale $V_o$ à l'instant où est commandée la logique 4 dans le sens du blocage et, pendant toute la période de fonctionnement de la régulation automatique, délivre un signal $V_o'$ fixe, égal à ce signal enregistré $V_o$.

Une fausse manoeuvre sur le bouton du potentiomètre 7 pendant cette période n'a aucun effet sur le signal $V_o'$ issu de la mémoire 8 et, donc, sur le signal $\mathcal{E}$ qui commande les moyens de pompage P.

En outre, l'ensemble potentiométrique ci-dessus évoqué comprend un comparateur 9 qui reçoit, d'une part, le signal $V_o$ issu du potentiomètre 7, d'autre part, le signal de sommation $\mathcal{E}$ issu du sommateur 6. Ce comparateur 9 est relié à sa sortie à la logique de blocage 4 pour délivrer vers celle-ci un signal fonction de la différence des signaux à ses deux entrées. Ce signal, désigné par signal d'inhibition $\Delta i$, est destiné à inhiber l'action de la commande manuelle de la logique 4 dans le cas où il possède une valeur différente de O ; à cet effet, la logique de blocage est adaptée pour permettre cette inhibition, par exemple par l'adjonction classique d'une porte à l'entrée de la commande manuelle.

Le comparateur 9 peut notamment être adapté pour délivrer un signal logique $\Delta i$ à trois niveaux :

. niveau 0 si la différence entre les signaux d'entrée est inférieure à un seuil déterminé ($|\mathcal{E} - v_o| < \mathcal{J}$)

. niveau 1 si cette différence est supérieure à ce seuil dans un sens (par exemple $V_o \geqslant \mathcal{E} + \mathcal{J}$ )

. niveau – 1 si cette différence est supérieure à ce seuil dans l'autre sens ($V_o \leqslant \mathcal{E} - \mathcal{J}$ )

Pour le niveau 0 qui correspond à un signal $V_o$ peu différent de $\mathcal{E}$ ($V_o \doteq \mathcal{E}$) la commande manuelle de la logique 4 n'est pas inhibée et agit pleinement. Elle permet en particulier de débloquer les mémoires 3 et 8 pour revenir, après une période de régulation automatique , à un réglage manuel des moyens de pompage P et ce, sans risque de variation brusque du signal $\mathcal{E}$ qui commande les moyens de pompage P, et donc sans risque de changement brusque du régime de ces moyens de pompage P ; en effet, le déblocage des mémoires 3 et 8 par commande de la logique 4 conditionne une annulation du signal $\Delta$ ( $\Delta = o$) et une égalisation du signal V' et du signal $V_o$ ($V'_o = V_o$) : en conséquence, le nouveau signal $\mathcal{E}$ issu du sommateur est égal à $V_o$ et se trouve donc peu différent du signal $\mathcal{E}$ existant immédiatement avant le déblocage.

Pour les niveaux –1 et +1 du signal $\Delta i$, la commande manuelle est inhibée, ce qui empêche un retour au réglage manuel des moyens de pompage P, retour qui, en raison d'une variation brusque du signal $\mathcal{E}$ , s'accompagnerait d'un changement brusque du régime des moyens de pompage P, susceptible de produire des traumatismes chez le malade.

Il faut noter que l'inhibition de la commande manuelle ne peut pas se produire à la fin de la période manuelle d'initialisation lorsque l'opérateur agit sur la logique 4 dans le sens du blocage pour passer en régime de régulation automatique ; en effet, pendant cette période manuelle, $\Delta = 0$ et $\mathcal{E} = V'_o = V_o$ ce qui condition-

ne $\Delta i = 0$.

En outre, des moyens de visualisation sont de préférence reliés au comparateur 9 pour afficher la présence et le sens du signal $\Delta i$.

Si $\Delta i = 0$, ces moyens demeurent éteints et la commande de la logique 4 produit son effet.

Si $\Delta i = -1$ une ampoule s'allume permettant de savoir que $V_o < \measuredangle$ : il convient alors d'actionner le potentiomètre pour augmenter $V_o$ jusqu'à obtenir un signal $\Delta i = 0$ qui autorise la commande de la logique 4.

Si $\Delta i = +1$, l'action inverse doit être exécutée sur le potentiomètre 7.

On conçoit donc l'intérêt de ces sécurités qui éliminent tout risque de traumatisme du malade et mettent celui-ci à l'abri des erreurs humaines ou fausses manoeuvres susceptibles de se produire.

Le dispositif de régulation décrit permet de réaliser la régulation autour d'une consigne choisie par l'opérateur et réglable dans chaque cas ; son processus de fonctionnement est résumé ci-après :

Au début des opérations, après branchement de la C.E.C. sur le malade, le dispositif de régulation se trouve en position manuelle (mémoire 3 et 8 non bloquées ); l'opérateur surveille le niveau du sang dans l'unité d'oxygénation et agit sur le potentiomètre 7 pour faire monter ou descendre celui-ci vers un niveau de consigne désiré. Lorsque ce niveau est atteint, il lui suffit de commander la logique 4 dans le sens du blocage des mémoires 3 et 8 : la régulation automatique autour de ce niveau de consigne est alors mise en fonctionnement.

Le signal détecté au moment du blocage, qui est représentatif de ce niveau de consigne, est enregistré dans la mémoire 3 qui le délivre à une entrée de comparateur 5. Si, par exemple, apparaît à un instant donné un excédent de sang dans l'unité d'oxygénation, qui fait monter le niveau, un signal d'écart $\Delta$ est délivrée par le comparateur 5 et module dans le sens de la croissance le signal $\measuredangle$ ; ce dernier entraîne une variation de la vitesse des moyens de pompage P dans le sens d'une accélération jusqu'à ramener le niveau de sang à coïncider avec le niveau consigne.

Il est possible dans ces conditions de réduire considérablement la quantité totale de sang contenue dans la C.E.C. ; en effet, la régulation réalise une régulation permanente et automatique du débit de sang injecté vers le malade, de sorte que la quantité de sang dans la C.E.C. demeure sensiblement constante, même avec une quantité de sang réduite dans la C.E.C. : on élimine ainsi tout risque d'insufflation de bulles d'air vers le malade, sans qu'il soit nécessaire d'opérer une surveillance particulière.

Pour accroître encore la sécurité, le dispositif de l'invention est équipé de moyens d'alarme en vue d'avertir l'opérateur de toute situation exceptionnelle :

. soit fonctionnement des moyens de pompage au régime maximum, ne permettant plus de réguler un débit plus grand provenant du malade,

. soit fonctionnement de ces moyens de pompage au régime minimum, risquant de provoquer leur arrêt,

. soit apparition d'un niveau de sang atteignant un seuil maximum,

. soit apparition d'un niveau de sang atteignant un seuil minimum.

Les deux première situations sont en fait indépendantes des deux dernières, mais se produisent généralement en combinaison avec celles-ci, avec éventuellement des temps de décalage. La détection de ces quatre situations fournit une sécurité redondante qui garantit que, dans chaque cas, l'opérateur sera averti suffisamment tôt pour pouvoir faire face à la situation et éviter des traumatismes au malade.

Les moyens d'alarme comprennent un organe 11 d'alarme visuelle et/ou sonore, commandé par un signal de basculement Aq ou par un signal de coïncidence Av.

Le signal Aq est engendré par un comparateur 12 qui reçoit, d'une part, le signal détecté par les moyens de détection 1, d'autre part, des signaux de consigne représentatifs d'un seuil minimum et d'un seuil maximum de la quantité de sang. A partir du franchissement de ces seuils par le signal issu des moyens de détection, le comparateur 12 délivre un signal de basculement Aq qui déclenche l'organe d'alarme 11.

De façon analogue, le signal Av est engendré par un comparateur 13 qui reçoit, d'une part, le signal $\Sigma$ issu du sommateur 6, d'autre part, des signaux de consigne représentatifs d'un seuil minimum et d'un seuil maximum de vitesse ; le seuil minimum peut correspondre à la vitesse la plus basse au-dessous de laquelle il existe des risques d'arrêt des moyens de pompage P, et le seuil maximum peut correspondre à la vitesse la plus élevée à laquelle peuvent tourner ces moyens. Lorsque le signal de sommation $\Sigma$ atteint ces seuils, le comparateur 13 délivre un signal de coïncidence Av qui déclenche l'organe d'alarme 11.

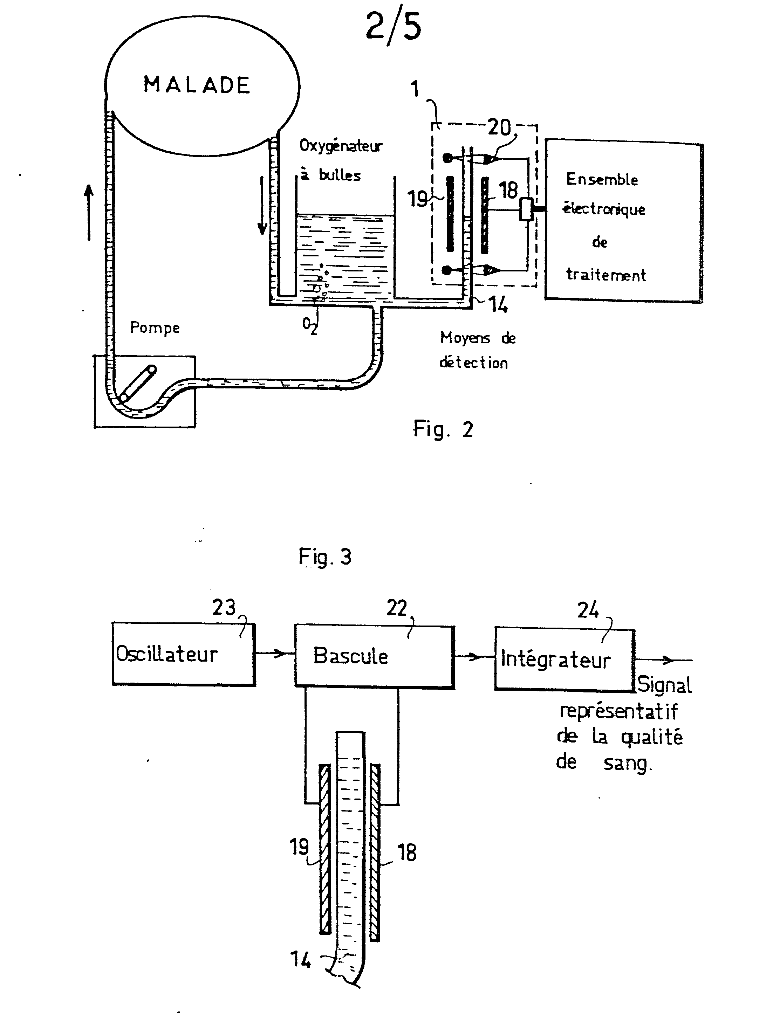

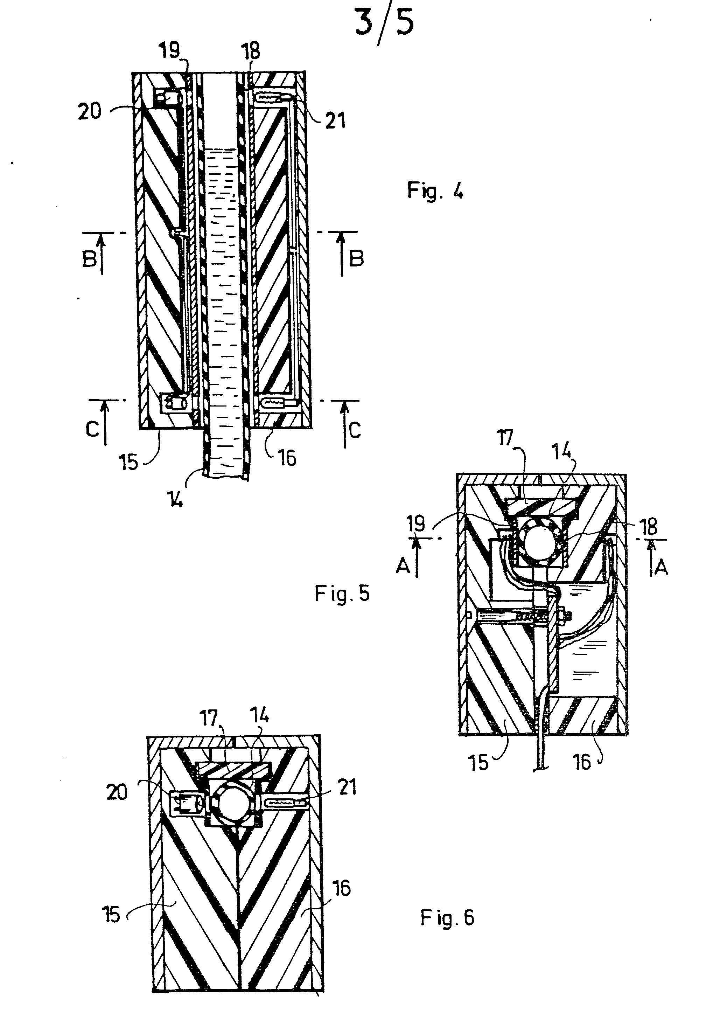

Les figures 2, 3, 4, 5 et 6 illustrent l'application de l'invention au cas d'une C.E.C. composée d'un oxygénateur à bulles et d'une pompe notamment à galets, telle que représentée à la figure 2.

Dans ce cas, les moyens de détection comprennent un tube auxiliaire 14 rempli de sérum physiologique et monté en vase communicant sur l'oxygénateur à bulles, comme l'illustrent les figures 2 à 6.

Ce tube 14 en matériau isolant électrique, par exemple en P.V.C., est maintenu dans un logement délimité entre deux platines en regard 15 et 16, s'étendant verticalement et fixées l'une sur l'autre. Un barrette 17 est glissée dans des rainures frontales de ces platines pour fermer le logement contenant le tube 14 en vue de bloquer ce tube. Les platines sont également en matériau isolant électrique.

Sur chaque platine 15 et 16, de part et d'autre du logement du tube 14 sont assujetties des armatures longiformes 18 et 19 d'un condensateur, s'étendant verticalement le long du tube 14, l'une d'un côté de ce tube, l'autre en regard de l'autre côté de celui-ci.

En outre, deux composants photosensibles tels que 20 associés à deux sources lumineuses telles que 21 sont disposés en partie basse et en partie haute des platines 15 et 16, dans le logement du tube. L'un des composants 20 est disposé en partie basse de l'armature 19 en regard du tube 14, avec sa source lumineuse 21 située de l'autre côté du tube 14, en face de celui-ci, en vue de l'éclairer. L'autre composant et sa source lumineuse ont un agencement analogue en partie haute/ des armatures du condensateur.

Comme le schématise la figure 3, les armatures 18 et 19 du condensateur sont électriquement reliées à une bascule monostable 22, excitée par un oscilla-teur d'alimentation 23 ; la bascule 22 délivre un signal ayant un rapport cyclique fonction de la capacité du condensateur. Ce signal est envoyé vers un intégrateur 24 qui délivre à son tour un signal continu d'amplitude fonction du rapport cyclique du signal issu de la bascule 22 ; ce signal continu est donc représentatif de la valeur de la capacité du condensateur (18,19). Cette capacité étant directement fonction du niveau de sérum contenu dans le tube 14 et, en conséquence de la quantité de sang contenue dans l'oxygénateur de la C.E.C.,le signal issu de l'intégrateur 24 constitue le signal de détection déjà évoqué, qui est représentatif de quantité de sang, et qui est envoyé vers l'ensemble de traitement déjà décrit.

Il convient de noter que, dans ce mode de réalisation, le sang n'a aucun contact direct avec les moyens de détection, et ne peut subir aucun traumatisme de la part de ceux-ci.

Les composants photosensibles 20 four-nissent des signaux de seuil lorsque le niveau de sérum physiologique s'abaisse au-dessous du composant inférieur, ou s'élève au-dessus du composant supérieur. L'enplacement de ces composants définit les seuils maximum et minimum de quantité de sang au-delà desquels l'alarme est déclenchée.

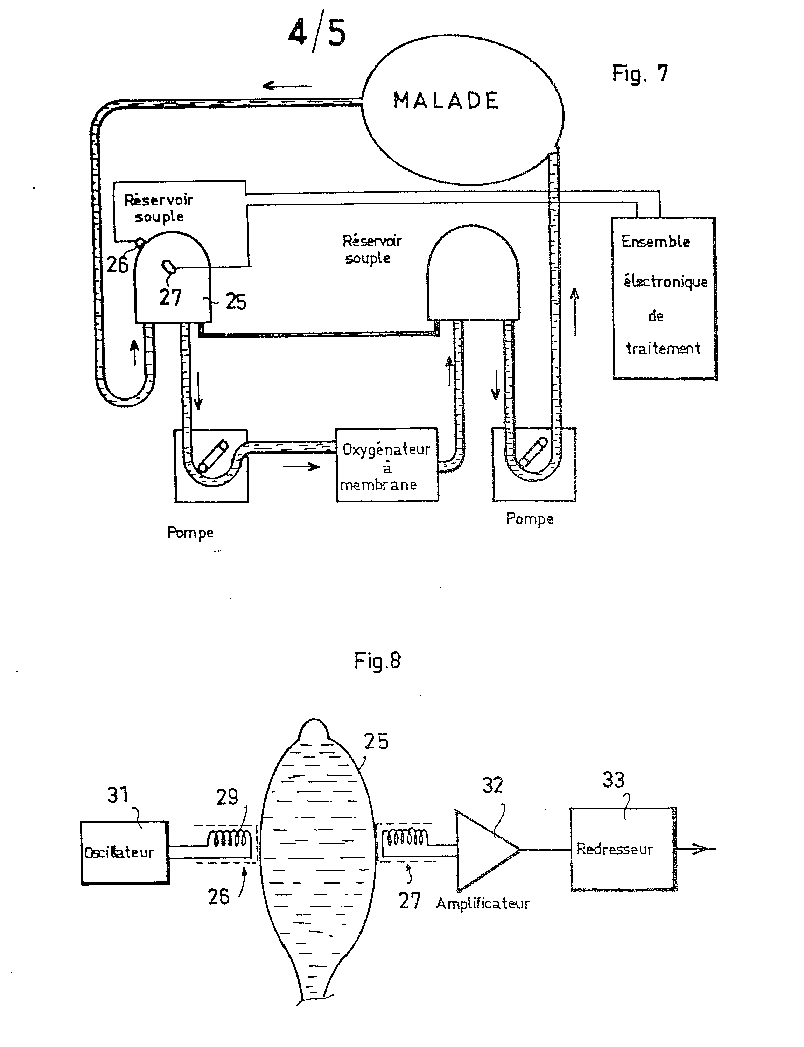

Les figures 7, 8 et 9 illustrent l'application de l'invention au cas d'une C.E.C. composée d'un oxygénateur à membrane, de deux réservoirs souples disposées en amont et en aval de cet oxygénateur pour régulariser le débit de sang dans celui-ci et de deux pompes notamment à galets (Fig. 7).

Dans ce cas, les moyens de détection sont associés à l'un des réservoirs souples 25 et compren-nent deux éléments inductifs 26 et 27 fixés en regard l'un de l'autre de part et d'autre du réservoir 25, à l'extérieur de celui-ci.

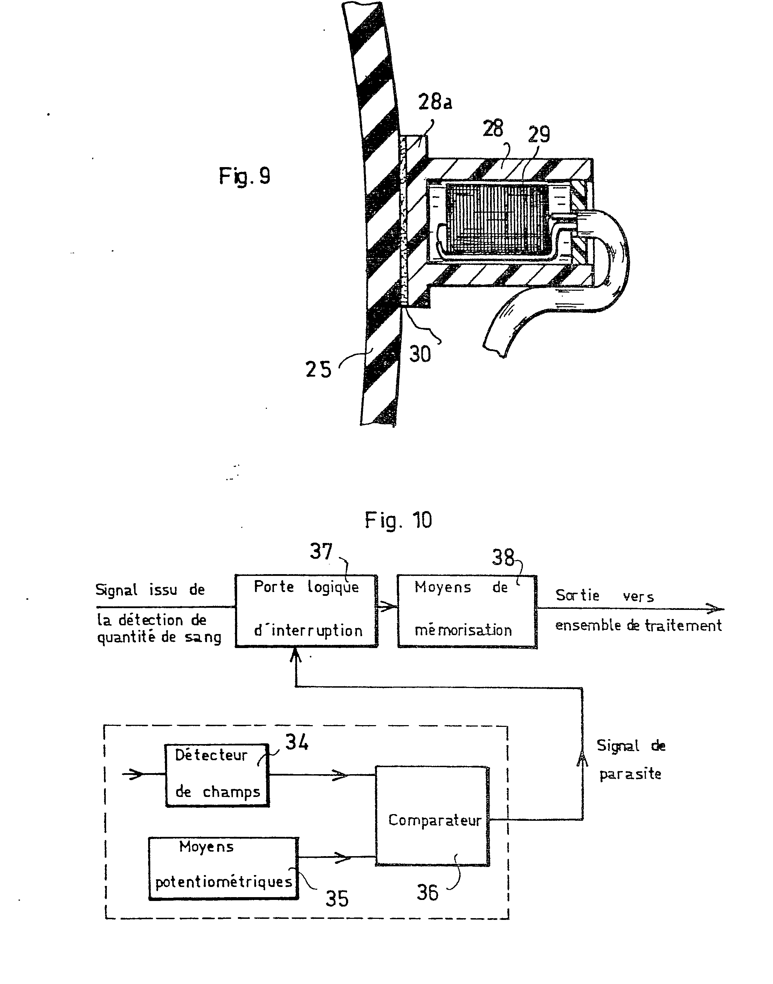

Chaque élément inductif est représenté en coupe axiale à la figure 9, et est composé d'un support 28 en matériau isolant électrique, d'un bobinage 29 maintenu par ledit support et d'une pastille autocollante 30, collée sur une collerette 28a que comporte le support à une extrémité. En l'exemple, le bobinage 29 est disposé dans un logement intérieur du support 28, qui est fermée par un disque d'obturation.

Un tel élément inductif est facile à fixer par collage sur la paroi du réservoir souple 25 de la C.E.C.

Comme le schématise la figure 8, le bobinage 29 de l'un des éléments inductifs 26 est électriquement relié à un oscillateur 31 en vue de son excitation, cependant que le bobinage de l'autre élément inductif 27 est relié à des moyens de mesure du signal induit, comprenant un amplificateur 32 et des moyens de redressement 33.

Dans ce mode de réalisation, la quantité de sang contenue dans l'unité d'oxygénation est représentée par le volume des réservoirs souples. Lorsque cette quantité augmente, le réservoir 25 gonfle : le signal induit dans l'élément 27 est fonction de ce gonflement, et, en conséquence, le signal issu des moyens de redressement 33 est représentatif de la quantité de sang dans la C.E.C.

Il est à noter que les très faibles énergies électromagnétiques mises en jeu au niveau des éléments inductifs ne font subir au sang contenu dans le réservoir 25 aucun risque de traumatisme.

Par ailleurs, il est fréquent dans les salles d'opération que des appareillages électriques créent momentanément des parasites HF qui pourraient perturber les signaux représentatifs de quantité de sang, issus des moyens de détection décrits précédemment.

Comme il a été indiqué, un circuit électronique d'immunité 2 est avantageusement associé aux moyens de détection pour éliminer l'influence préjudiciable de ces perturbations.

La figure 10 est un schéma fonctionnel, illustrant un exemple de circuit d'immunité.

Ce circuit comprend un ensemble de détection des champs parasites, composé d'un détecteur de champs 34 ( diode redresseuse polarisée ), de moyens potentiométriques 35 qui fournissent un signal réglable définissant le seuil de déclenchement du circuit, et d'un comparateur 36 qui reçoit le signal détecté et le signal de seuil et délivre un signal de parasite, notamment à 2 niveaux, selon que le signal détecté est inférieur ou supérieur au signal de seuil.

Cet ensemble de détection (34, 35, 36) est relié à une porte logique 37, dite porte logique d'interruption, qui est disposée en série, en aval des moyens de détection de la quantité de sang.. Cette porte logique d'interruption 37 est adaptée pour se fermer lorsque le signal issu du comparateur 36 présente une valeur indicative de la présence de parasites, afin d'interrompre la transmission du signal issu des moyens de détection.

Des moyens de mémorisation 38 sont associés à la porte logique d'interruption 37 ; ces moyens sont adaptés pour transmettre le signal issu de cette porte lorsque celle-ci est passante ( porte ouverte ) , et pour maintenir la transmission du dernier signal reçu en cas de fermeture de la porte.

Un tel circuit d'immunité ne supprime pas les parasites mais, en cas d'apparition d'une perturbation supérieure à un seuil ajustable, il isole les moyens de détection de l'ensemble de traitement et fournit à ce dernier un signal égal au dernier signal reçu avant la perturbation : celle-ci n'a donc aucune influence sur les moyens de pompage P, dont la vitesse est maintenue pendant la perturbation.

Ces moyens de mémorisation 38 peuvent être analogiques, et formés par un condensateur connecté à la sortie de la porte logique d'interruption pour se charger lorsque cette porte est ouverte, et un transistor de décharge disposé en parallèle du condensateur pour permettre à celui-ci de se décharger de sorte que la charge accumulée soit à chaque instant représentative du signal issu de la porte logique 37 lorsque cette dernière est ouverte.

Ce transistor est agencé pour se bloquer lorsque la porte logique 37 se ferme , et la charge du condensateur est alors conservée. Une telle mémoire possède une durée limitée dans le temps ( $10^n$ environ ), mais celle-ci est amplement suffisante, compte tenu de la courte durée des parasites.

Bien entendu, l'invention n'est pas limitée aux termes de la description précédente, mais en comprend toutes les variantes.

REVENDICATIONS

1/ - Dispositif de régulation du débit de sang délivré vers un malade par un système de circulation extracorporelle composé de moyens de conduite du sang comportant un conduit amont de prélèvement du sang du malade et un conduit aval d'injection du sang vers le malade, de moyens de pompage engendrant une circulation du sang du conduit amont de prélèvement vers le conduit aval d'injection et d'une.unité d'oxygénation du sang placée entre le conduit amont de prélèvement et le conduit aval d'injection, ledit dispositif de régulation étant caractérisé en ce qu'il comprend :

. des moyens de détection de la quantité de sang contenue à chaque instant dans l'unité d'oxygénation, adaptés pour délivrer un signal électrique représentatif à chaque instant de cette quantité de sang,

. une mémoire, dite de consigne, recevant le signal issu des moyens de détection et associée à une logique de blocage permettant de figer, à un instant donné, le signal reçu dans la mémoire,

. un comparateur, recevant, d'une part, le signal issu des moyens de détection, d'autre part, le signal issu de la mémoire de consigne et adapté pour délivrer un signal d'écart représentatif de la différence entre les signaux reçus,

. un ensemble potentiométrique adapté pour délivrer un signal de vitesse correspondant à une vitesse initiale désirée des moyens de pompage,

. un sommateur recevant le signal d'écart issu du comparateur et le signal de vitesse issu de l'ensemble potentiométrique et adapté pour délivrer vers les moyens de pompage, un signal d'asservissement approprié pour accélérer lesdits moyens de pompage en cas d'augmentation de la quantité de sang détectée et pour ralentir ces moyens de pompage en cas de baisse de la quantité de sang détectée.

2/ - Dispositif de régulation selon la revendication 1, caractérisé en ce que la logique de blocage associée à la mémoire de consigne possède une commande manuelle permettant à un opérateur de figer ladite mémoire.

3/ - Dispositif de régulation selon l'une des revendications 1 ou 2, caractérisé en ce que l'ensemble

potentiométrique comprend un potentiomètre pour engendrer un signal de valeur réglable et une mémoire dite de sécurité, recevant ce signal et reliée à sa sortie au sommateur, ladite mémoire de sécurité étant reliée à la logique de blocage sus-évoquée en vue d'être figée en même temps que la mémoire de consigne.

4/ - Dispositif de régulation selon la revendication 3, caractérisé en ce que l'ensemble potentiométrique comprend un comparateur recevant le signal issu du sommateur et le signal issu du potentiomètre . et délivrant un signal fonction de la différence de ces signaux vers la logique de blocage sus-évoquée, ladite logique de blocage possédant une commande manuelle et étant adaptée pour inhiber l'action de cette commande dans le cas où le signal issu du comparateur est différent de 0.

5/ - Dispositif de régulation selon la revendication 4, caractérisé en ce que des moyens de visualisation sont reliés au comparateur précité, ces moyens étant aptes à afficher la présence et le sens du signal issu du comparateur.

6/ - Dispositif de régulation selon l'une des revendications 1, 2, 3, 4 ou 5, comprenant des moyens d'alarme adaptés pour se déclencher dans le cas où la quantité de sang dans l'unité d'oxygénation sort d'une plage délimitée par des seuils minimum et maximum déterminés, ledit dispositif étant caractérisé en ce que ces moyens d'alarme comprennent un comparateur recevant, d'une part, le signal issu des moyens de détection, d'autre part, des signaux de consigne représentatifs des seuils minimum et maximum sus-évoqués, en vue de délivrer un signal de basculement à partir du franchissement de ces seuils par le signal issu des moyens de détection, et un organe d'alarme visuelle et/ou sonore, commandé par le signal de basculement issu du comparateur.

7/ - Dispositif de régulation selon l'une des revendications 1, 2, 3, 4, 5 ou 6, caractérisé en ce qu'il comprend des moyens d'alarme adaptés pour se déclencher dans le cas où la vitesse des moyens de pompage atteint des seuils minimum ou maximum déterminés, ces moyens d'alarme comprenant un comparateur recevant, d'une part, le signal issu du sommateur, d'autre part, des signaux de consigne représentatifs des seuils minimum et maximum sus-évoqués en vue de délivrer un signal de coïncidence lorsque le signal de sommation atteint ces seuils, et un organe d'alarme visuelle et/ou sonore commandé par le signal de coïncidence issu du comparateur.

8/ - Dispositif de régulation selon l'une des revendications 1, 2, 3, 4, 5, 6 ou 7, dans lequel l'unité d'oxygénation est composée d'un oxygénateur à bulles, les moyens de détection de la quantité de sang comprenant un tube auxiliaire en matériau isolant électrique monté en vase communicant sur l'oxygénateur, un condensateur ayant des armatures situées en regard de part et d'autre du tube auxiliaire, et des moyens de mesure de la capacité dudit condensateur délivrant le signal représentatif de la quantité de sang dans l'oxygénateur.

9/ - Dispositif de régulation selon la revendication 8, caractérisé en ce que le tube auxiliaire contient du sérum physiologique.

10/ - Dispositif de régulation selon l'une des revendications 8 ou 9, dans lequel les moyens de mesure de la capacité du condensateur comprennent une bascule monostable excitée par un oscillateur d'alimentation et connectée aux armatures du condensateur en vue de délivrer un signal ayant un rapport cyclique fonction de la capacité du condensateur, et un intégrateur recevant ledit signal et adapté pour délivrer un signal continu d'amplitude fonction du rapport cyclique du signal issu de la bascule monostable.

11/ - Dispositif de régulation selon les revendications 6 et 7 prises ensemble, dans lequel les moyens de détection comprennent deux composants photosensibles associés à deux sources lumineuses, l'un des composants étant disposé en partie basse des armatures du condensateur en regard du tube auxiliaire avec sa source lumineuse située à l'autre côté du tube en face dudit composant, l'autre composant et sa source lumineuse ayant un agencement analogue, en partie haute des armatures du condensateur.

12/ - Dispositif de régulation selon l'une des revendications 1, 2, 3, 4, 5, 6, 7 ou 8, dans lequel l'unité d'oxygénation est composée d'un oxygénateur à membrane et d'au moins un reservoir souple de sang disposé en amont et/ou en aval de l'oxygénateur à membrane pour régulariser le débit de sang dans ledit oxygénateur à membrane, le dispositif de régulation étant caractérisé en ce que les moyens de détection de la quantité de sang comprennent deux éléments inductifs fixés en regard l'un de l'autre, de part et d'autre du réservoir souple à l'extérieur de celui-ci, un oscillateur électrique relié à l'un des éléments inductifs en vue de son excitation et des moyens de mesure du signal induit dans l'autre élément inductif..

13/ - Dispositif de régulation selon la revendication 12, caractérisé en ce que chaque élément inductif est collé sur la paroi externe souple du réservoir au moyen d'une face autocollante que comprend cet élément inductif.

14/ - Dispositif de régulation selon la revendication 13, caractérisé en ce que chaque élément inductif comprend un bobinage maintenu par un support en matériau isolant électrique lequel se termine par une collerette possédant une face autocollante.

15/ - Dispositif de régulation selon l'une des revendications 12, 13 ou 14, dans lequel les moyens de mesure du signal induit comprennent un amplificateur recevant ce signal induit et des moyens de redressement délivrant le signal représentatif de la quantité de sang.

16/ - Dispositif de régulation selon l'une des revendications 8, 9, 10, 11, 12, 13, 14 ou 15, caractérisé en ce qu'il comprend un circuit électronique dit d'immunité, disposé immédiatement après les moyens de détection de la quantité de sang et comportant :

. un ensemble de détection de champs électrique parasites, adapté pour délivrer un signal électrique dit signal de parasite lorsque le champ détecté dépasse un seuil déterminé,

. une porte logique d'interruption, disposée en série en aval des moyens de détection de la quantité de sang, cette porte logique étant reliée à l'ensemble de détection de champs parasites pour recevoir le signal de parasite et étant adaptée pour se fermer et interrompre la transmission du signal issu des moyens de détection de la quantité de sang en présence dudit signal de parasite,

. des moyens de mémorisation disposés à la

**0030760**

sortie de la porte logique et adaptés pour transmettre le signal issu de celle-ci lorsque ladite porte logique est ouverte et pour maintenir la transmission du dernier signal reçu en cas de fermeture de la porte.

17/ - Dispositif de régulation selon la revendication 16, caractérisé en ce que les moyens de mémorisation sus-évoqués sont analogiques et comprennent un condensateur connecté à la sortie de la porte logique d'interruption pour se charger lorsque cette porte est ouverte, et un transistor de décharge disposé en parallèle du condensateur pour permettre à celui-ci de se décharger de sorte que la charge accumulée soit à chaque instant représentative du signal issu de la porte logique lorsque cette dernière est ouverte.

18/ - Moyen de détection d'une quantité de liquide emmagasinée dans un réservoir souple, spécialement conçu pour équiper le dispositif de régulation selon la revendication 12, caractérisé en ce qu'il comprend deux éléments inductifs adaptés pour être fixés en regard l'un de l'autre, de part et d'autre du réservoir souple, un oscillateur électrique relié à l'un des éléments inductifs en vue de son excitation, et des moyens de mesure du signal induit dans l'autre élément inductif.

19/ - Elément inductif appelé à équiper des moyens de détection conformes à la revendication 18, caractérisé en ce qu'il comprend un support en matériau isolant électrique, un bobinage maintenu par ledit support et une collerette disposée en bout dudit support, et dotée d'une face adaptée pour permettre la fixation de l'élément.

Fig.1

Consigne quantité mini
Consigne quantité maxi

Comparateur 12

Aq

Alarme 11

Av

Comparateur 13

Consigne vitesse mini
Consigne vitesse maxi

Vers le malade — Sang Cp

Unité d'oxygénation

C.E.C.

P

Moyens de détection

Circuit d'immunité 1

2

Mémoire de consigne 3

Comparateur 5

Δ

Commande

Logique de blocage 4

Inhibition commande

Δi

10

Comparateur 9

Sommateur 6

W

8 Mémoire de sécurité

V'o

Potentiomètre 7

Vo

1/5

0030760

MALADE

Oxygénateur
à bulles

Ensemble
électronique
de
traitement

1

20

19

18

14

$O_2$

Pompe

Moyens de
détection

**Fig. 2**

**Fig. 3**

| Oscillateur | → | Bascule | → | Intégrateur | → |
|---|---|---|---|---|---|

23

22

24

Signal
représentatif
de la qualité
de sang.

19

18

14

0030760

3/5

Fig. 4

Fig. 5

Fig. 6

0030760

Fig. 7

MALADE

Réservoir
souple

26

27    25

Réservoir
souple

Ensemble
électronique
de
traitement

Oxygénateur
à
membrane

Pompe

Pompe

Fig.8

31

29

25

32

33

Oscillateur

26

27    Amplificateur

Redresseur

5/5

Fig. 9

28a

28  29

25  30

Fig. 10

Signal issu de
la détection de
quantité de sang

| Porte logique | 37 |
| d'interruption |

| Moyens de | 38 |
| mémorisation |

Sortie vers
ensemble de traitement

Signal de
parasite

| Détecteur | 34 |
| de champs |

| Moyens | |
| potentiométriques | 35 |

| Comparateur | 36 |

**0030760**

Numéro de la demande

Office européen
des brevets

## RAPPORT DE RECHERCHE EUROPEENNE

EP 80 20 1148

| | DOCUMENTS CONSIDERES COMME PERTINENTS | | CLASSEMENT DE LA DEMANDE (Int. Cl. 3) |
|---|---|---|---|
| Catégorie | Citation du document avec indication, en cas de besoin, des parties pertinentes | Revendication concernée | |
| | <u>US - A - 3 851 181</u> (HEULE)<br><br>  * figures 1,2A,2B,3,7; colonne 2, ligne 16 à colonne 3, ligne 6; colonne 3, ligne 28 à colonne 6, ligne 10; colonne 10, lignes 9-61 *<br><br>-- | 1,2,6, 11 | A 61 M 1/03<br>G 05 D 9/12 |
| | <u>FR - A - 2 053 869</u> (ORTOLAN)<br><br>  * figures 1-4; page 1, lignes 1-3; page 1, ligne 23 à fin page 2 *<br><br>-- | 1,6,8, 10 | |
| | | | DOMAINES TECHNIQUES RECHERCHES (Int. Cl. 3) |
| | <u>US - A - 3 513 845</u> (CHESNUT, <u>CALLAGHAN, GAFVERT</u>)<br><br>- - - - - - - - - | 1 | A 61 M<br>G 05 D |

CATEGORIE DES DOCUMENTS CITES

X: particulièrement pertinent

A: arrière-plan technologique

O: divulgation non-écrite

P: document intercalaire

T: théorie ou principe à la base de l'invention

E: demande faisant interférence

D: document cité dans la demande

L: document cité pour d'autres raisons

&: membre de la même famille, document correspondant

X Le présent rapport de recherche a été établi pour toutes les revendications

| Lieu de la recherche | Date d'achèvement de la recherche | Examinateur |
|---|---|---|
| La Haye | 19-3-1981 | HERBELET |

OEB Form 1503.1 06.78